# EUROPÄISCHE PATENTSCHRIFT

(11) **EP 0 846 100 B1**
(45) Veröffentlichungstag und Bekanntmachung des Hinweises auf die Patenterteilung: **16.02.2000**
(21) Anmeldenummer: 96928421.5
(22) Anmeldetag: 07.08.1996
(51) Int. Cl.: C07C 233/36, C07C 233/38

(54) **AMIDOPROPYL-N-ALKYL-POLYHYDROXYALKYLAMIN-VERBINDUNGEN, VERFAHREN ZU DEREN HERSTELLUNG UND DEREN VERWENDUNG**
AMINOPROPYL-N-ALKYL-POLYHYDROXY ALKYL AMINE COMPOUNDS, PROCESS FOR THEIR PRODUCTION AND THEIR USE
COMPOSES AMINOPROPYL-N-ALKYLPOLYHYDROXYALKYLAMINES, LEUR PROCEDE DE PRODUCTION ET LEUR UTILISATION

(30) Priorität: 14.08.1995 DE 19529810
(43) Veröffentlichungstag der Anmeldung: 10.06.1998
(73) Patentinhaber: Clariant GmbH, 65926 Frankfurt am Main (DE)
(72) Erfinder: PROSSEL, Günter, D-84508 Burgkirchen (DE); VYBIRAL, Reinhard, D-84508 Burgkirchen (DE); SKRYPZAK, Werner, D-65719 Hofheim (DE); SCHOLZ, Hans, Jürgen, D-63755 Alzenau (DE)
(86) Internationale Anmeldenummer: EP9603486
(87) Internationale Veröffentlichungsnummer: WO9707090

(56) Entgegenhaltungen:
- WO-A-95/32942
- DE-C- 4 238 211

## Beschreibung

In DE-A-42 38 207 und in DE-C-42 38 211 werden quaternisierte Fettsäurepolyhydroxyalkylamide beschrieben, die sich formal von Propylendiaminen ableiten. Diese kationischen Tenside sollen eine hohe Wasserlöslichkeit aufweisen und sich zu hochkonzentrierten, niedrigviskosen Lösungen oder Dispersionen verarbeiten lassen.

Im Beispiel 1 der DE-A-42 38 207 und im Beispiel Ea) der DE-C-42 38 211 wird gleichlautend die reduktive Aminierung von Glucose und N,N'-Dimethyl-ethylendiamin beschrieben, wobei die beiden Komponenten im Molverhältnis 1 : 1 eingesetzt werden. Diese Mischung wird in methanolischer Lösung mit Raney-Nickel hydriert. In beiden Schriften wird angegeben, daß der Aminozucker in Form eines weißen Stoffes in praktisch quantitativer Ausbeute gewonnen wurde. Man erkennt jedoch sofort, daß die Reaktion weitgehend statistisch ablaufen wird, das heißt, es wird ein Produktgemisch entstehen, welches N-Monoglucosid, N,N'-Diglucosid und nichtumgesetztes Ausgangsamin enthält. Die Aussage "in praktisch quantitativer Ausbeute" bezieht sich offenbar nur auf die Massenbilanz, nicht aber auf die chemische Zusammensetzung. Signifikanterweise werden weder in DE-A-42 38 207 noch in DE-C-42 38 211 keinerlei analytische Untersuchungen angegeben, welche die vorgebliche Einheitlichkeit des Produkts bestätigen könnten. An geeigneten Methoden, beispielsweise HPLC, war zum Zeitpunkt der Einreichung der DE-A-42 38 207 und DE-C-42 38 211 sicher kein Mangel. Im Unterschied zur Lehre der DE-A-42 38 207 und DE-C-42 38 211 führt das Verfahren gemäß der vorliegenden Erfindung zu sehr reinen Produkten.

Gegenstand der vorliegenden Erfindung ist somit ein verbessertes Verfahren zur Herstellung reiner Produkte der nachstehend-gezeigten Formeln (1) und (1a).

Die erfindungsgemäß hergestellten Amidopropyl-N-alkyl-polyhydroxyalkylamine entsprechen der nachstehenden Formel (1) 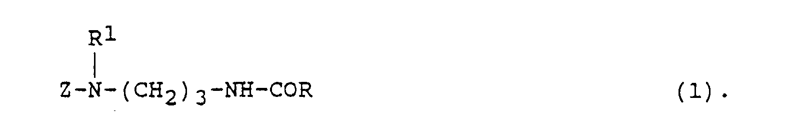 Hierin ist
- Z: ein linearer Polyhydroxykohlenwasserstoffrest mit mindestens 2 gegebenenfalls oxalkylierten Hydroxylgruppen,
- R¹: ein C₁ bis C₇-Alkylrest oder C₂ bis C₄-Hydroxyalkylrest und
- COR: ein aliphatischer Acylrest mit 6 bis 22 Kohlenstoffatomen.

Bevorzugte erfindungsgemäße Verbindungen der Formel (1) sind solche, bei denen
- Z: ein Polyhydroxyalkylrest mit 3 bis 18 Kohlenstoffatomen und 2 bis 13 Hydroxylgruppen ist und vorzugsweise ein Rest eines Zuckeralkohols ist, der sich von einem reduzierenden Mono- oder Disaccharid, insbesondere von Glucose, ableitet,
- R¹: Methyl, Ethyl, Propyl, Isopropyl oder -CH₂CH₂OH ist und
- COR: ein Fettacylrest mit 8 bis 18 Kohlenstoffatomen ist.

Zu COR und Z sei noch folgendes gesagt: Der aliphatische Acylrest COR, der vorzugsweise der genannte Fettacylrest ist, kann gesättigt oder ungesättigt (vorzugsweise ein- bis dreifach ungesättigt) sein. Als Beispiele seien die Acylreste von Capryl-, Caprin-, Laurin-, Palmitin-, Stearin- und Ölsäure genannt sowie Cocosacyl, Talgacyl, vorzugsweise gehärtetes Talgacyl, und dergleichen. Der Fettsäurerest stellt häufig eine Mischung von zwei oder mehreren Acylgruppen dar, zum Beispiel C₁₂ und C₁₄-Acyl (C_{12/14}), C₁₆ und C₁₈-Acyl (C_{16/18}) oder C₁₂ bis C₁₈-Acyl. Der lineare Polyhydroxykohlenwasserstoffrest stammt, wie oben bereits erwähnt, vorzugsweise von Zuckeralkoholen, abgeleitet von der Gruppe der reduzierenden Zucker oder reduzierenden Zuckerderivate. Bevorzugte reduzierende Zucker sind die Monosaccharide, vorzugsweise Pentosen und Hexosen, und die Oligosaccharide, vorzugsweise Disaccharide und gegebenenfalls auch Trisaccharide. Beispiele für Monosaccharide sind Glucose, Galaktose, Mannose und Talose als Hexosen und Arabinose, Ribose und Xylose als Pentosen. Von den Monosacchariden sind die Hexosen bevorzugt. Beispiele für Oligosaccharide (Polysaccharide) sind Lactose, Maltose, Maltotriose und dergleichen. Besonders bevorzugte Polyhydroxyalkylreste stammen von reduzierenden Hexosen, insbesondere von Glucose (Sorbitylrest).

Die Salze von einem erfindungsgemäß hergestellten Polyhydroxyalkylamin der Formel (1) mit einer anorganischen oder organischen Säure leiten sich im allgemeinen von einer Mineralsäure oder von einer aliphatischen oder aromatischen Carbonsäure oder Hydroxycarbonsäure ab. Bevorzugte anorganische Säuren sind Halogenwasserstoffsäuren wie HCl oder HBr, Kohlensäure, Phosphorsäure oder Schwefelsäure. Bevorzugte organische Säuren sind C₁ bis C₃-Monocarbonsäuren wie Ameisensäure, Essigsäure oder Propionsäure; C₁₂ bis C₁₈-Monocarbonsäure wie Dodecansäure, Palmitinsäure, Stearinsäure oder Ölsäure; Dicarbonsäuren der Formel HOOC-(CH₂)ₙ-COOH, worin n 0 oder eine ganze Zahl von 1 bis 8 ist, wie Oxal-, Malon-, Bernstein-, Glutar- oder Adipinsäure und die ungesättigten Dicarbonsäuren, Fumar- und Maleinsäure; Monohydroxy-monocarbonsäuren wie Glykolsäure (Hydroxyessigsäure) oder Milchsäure (a-Hydroxy-propionsäure); Monohydroxy-dicarbonsäuren wie Tartronsäure (Monohydroxy-malonsäure) oder Äpfelsäure (Monohydroxybernsteinsäure); Dihydroxy-dicarbonsäuren wie Weinsäure (Dihydroxy-bernsteinsäure); Monohydroxy-tricarbonsäuren wie Citronensäure (Hydroxy-tricarballylsäure); Hydroxybenzoesäuren wie Salicylsäure oder Gallussäure; und aromatische Dicarbonsäuren (Phthalsäuren) wie o-Phthalsäure oder p-Phthalsäure. Von den genannten organischen Säuren als Anionbildner sind die C₁ bis C₃-Monocarbonsäuren, die Milchsäure und die Fruchtsäuren besonders bevorzugt.

Formelmäßig entsprechen die Salze der nachstehenden Formel (1a) 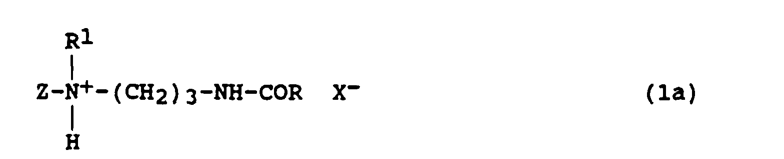 worin Z, R¹ und COR die angegebenen Bedeutungen haben und X⁻ ein Anion einer anorganischen oder organischen Säure ist.

Für den Fall, daß X⁻ das Anion einer mehrbasigen anorganischen oder organischen Säure ist, können die entsprechenden sauren Salze auch mit der äquivalenten Menge anorganischer oder organischer basischer Stoffe teilweise oder vollständig neutralisiert werden. Das saure Salz kann aber auch mit der entsprechend äquivalenten Menge einer Amidopropyl-N-alkylpolyhydroxyalkylamin- oder N-Alkyl-polyhydroxyalkylamin-Verbindung teilweise oder vollständig neutralisiert werden.

Ausgehend von den genannten Säuren seien zur weiteren Erläuterung einige Beispiele für X⁻ angeführt: Cl⁻, Br⁻, (SO₄²⁻)_{1/2}, (PO₄³⁻)_{1/3}, CH₃COO⁻, C₁₁H₂₃COO⁻, CH₃-CH(OH)-COO⁻ (Anion der Milchsäure), HOOC-CH(OH)-CH₂-COO⁻ (Anion der Äpfelsäure), C₆H₅-COO⁻ (Anion der Benzoesäure) und dergleichen.

Die erfindungsgemäßen Amidopropyl-N-alkylpolyhydroxyalkylamine der Formel (1) werden hergestellt durch
a) Umsetzung einer Verbindung der Formel (2) 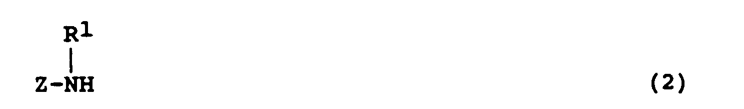 in der Z und R¹ die genannten Bedeutungen haben,
   mit Acrylnitril (CH₂=CH-CN) in einem polaren Lösungsmittel bei einer Temperatur von 50 bis 100 °C zur Bildung einer Verbindung der Formel (3) 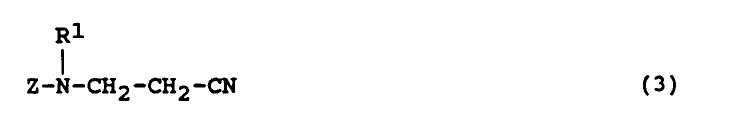 in der Z und R¹ die genannten Bedeutungen haben,
b) Umsetzung des im Schritt a) erhaltenen Produktes mit Wasserstoff in Gegenwart von Ammoniak und einem Hydrierkatalysator bei einer Temperatur von 20 bis 80 °C und einem Wasserstoffdruck von 10 bis 80 bar zur Bildung einer Verbindung der Formel (4) 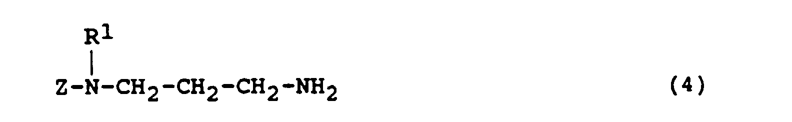 in der Z und R¹ die genannten Bedeutungen haben,
   und
c) Monoacylierung des im Schritt b) erhaltenen Produktes in der Schmelze mit einer vom Acylrest COR in Formel (1) abgeleiteten Säure oder Säurederivat in Gegenwart eines alkalischen Katalysators bei einer Temperatur von 80 bis 130 °C zur Bildung einer Verbindung der genannten Formel (1).

Das erfindungsgemäße Verfahren besteht also aus einer Kombination von mehreren speziellen Umsetzungen. Im folgenden werden bevorzugte Ausführungen der einzelnen Verfahrensstufen beschrieben.

Die Acrylnitril-Addition im Schritt a) wird in Gegenwart eines polaren Lösungsmittels bei einer Temperatur von vorzugsweise 60 bis 90 °C durchgeführt. Die Reaktionskomponente, das ist eine Verbindung der Formel (2), zum Beispiel N-Methylglucamin (im Handel erhältlich), und Acrylnitril werden vorzugsweise im Molverhältnis von etwa 1 : 1 eingesetzt. Geeignete polare Lösungsmittel sind C₁ bis C₄-Alkanole wie Methanol, Ethanol, Propanol oder Isopropanol, C₂ bis C₄-Diole wie Monoethylenglykol, Diethylenglykol, Monopropylenglykol oder Dipropylenglykol und die Mono- oder Diether dieser Diole wie Monoethylenglykolmonomethylether und dergleichen. Es kann auch eine Mischung der genannten Flüssigkeiten eingesetzt werden. Bevorzugte Lösungsmittel sind Methanol, Ethanol oder Isopropanol. Das Lösungsmittel wird in einer solchen Menge eingesetzt, daß eine vorzugsweise 10 bis 50gew.%ige Reaktionsmischung vorliegt. Die Umsetzung wird im allgemeinen bei Atmosphärendruck oder dem sich einstellenden Systemdruck durchgeführt und verläuft bei einer Reaktionszeit von etwa 1 bis 3 Stunden quantitativ. Das erhaltene Produkt besteht im wesentlichen aus einer Verbindung der genannten Formel (3) und dem eingesetzten Lösungsmittel. Die Abtrennung des Lösungsmittels und Gewinnung der Verbindung als solcher ist für die weitere Umsetzung nicht erforderlich.

Im Schritt b) wird die im Schritt a) erhaltene Lösung zur Überführung von -CN in -CH₂NH₂ mit Wasserstoff bei einer Temperatur von vorzugsweise 30 bis 60 °C und einem Druck von vorzugsweise 30 bis 60 bar umgesetzt. Es sind etwa 2 mol Wasserstoff pro mol Verbindung der Formel (3) erforderlich. Die Umsetzung wird in Gegenwart von Ammoniak und einem Hydrierkatalysator durchgeführt. Die Menge an Ammoniak zur Reduzierung von Nebenproduktbildungen liegt im allgemeinen bei 1 bis 10 mol, vorzugsweise 3 bis 6 mol, pro mol Nitrilverbindung der Formel (3). Es können die üblichen Hydrierkatalysatoren wie Kobalt, Kupfer, Eisen, Nickel, Palladium und/oder Platin eingesetzt werden, wobei Nickelkatalysatoren bevorzugt sind. Der metallische Hydrierkatalysator kann als Trägerkatalysator, Pulverkatalysator oder als Raney-Katalysator eingesetzt werden. Raney-Nickel ist bevorzugt. Der Hydrierkatalysator wird im allgemeinen in einer Menge von 0,05 bis 5 Gew.-% eingesetzt, vorzugsweise in einer Menge von 0,1 bis 2 Gew.-%, bezogen auf die zu hydrierende Nitrilverbindung (die genannten Gewichtsprozente beziehen sich auf das Metall allein, umfassen also nicht zum Beispiel auch das Trägermaterial). Die Hydrierung zur Verbindung der Formel (4) verläuft bei einer Reaktionszeit von etwa 2 bis 6 Stunden praktisch quantitativ. Das erhaltene Produkt besteht also im wesentlichen aus einer Verbindung der Formel (4) und dem im Schritt a) eingesetzten Lösungsmittel. Da die weitere Umsetzung (Acylierung) in der Schmelze, das heißt in Abwesenheit irgendeines Lösungsmittel durchzuführen ist, wird die Verbindung der Formel (4) aus der im Schritt b) erhaltenen Lösung gewonnen. Dies kann zum Beispiel durch Abdestillieren des Lösungsmittels bei einer Temperatur von vorzugsweise 40 bis 60 °C erfolgen, gegebenenfalls unter Anwendung eines Vakuums von etwa 10 bis 50 mbar (Wasserstrahlvakuum), nachdem vorher der Hydrierkatalysator abfiltriert worden ist.

Im Schritt c) des erfindungsgemäßen Verfahrens wird die im Schritt b) erhaltene Verbindung der Formel (4) in der Schmelze bei einer Temperatur von vorzugsweise 90 bis 120 °C in Gegenwart eines basischen Katalysators monoacyliert (amidiert). Das Acylierungsmittel, das man im allgemeinen in einer Menge von 1 bis 1,1 mol, vorzugsweise 1,01 bis 1,05 mol pro mol Verbindung der Formel (4) einsetzt, ist vorzugsweise eine Fettsäure oder ein Fettsäurederivat wie Fettsäurehalogenid oder Fettsäure-C₁ bis C₄-alkylester, abgeleitet vom Acylrest COR in Formel (1). Das bevorzugte Acylierungsmittel ist ein Fettsäure-C₁ bis C₄-alkylester, wobei ein Fettsäuremethylester besonders bevorzugt ist. Der basische Acylierungskatalysator ist vorzugsweise ein Alkalimetallhydroxid wie Natrium- oder Kaliumhydroxid oder ein Alkalimetall-C₁ bis C₄-alkoholat wie Natriummethylat. Die Menge an basischem Katalysator liegt bei 0,01 bis 0,2 mol, vorzugsweise 0,05 bis 0,1 mol pro mol Verbindung der Formel (4). Die Acylierung wird bei Atmosphärendruck oder vorzugsweise unter Anwendung eines Vakuums durchgeführt, womit eine schnellere Entfernung der entstehenden flüchtigen Anteile wie zum Beispiel Methanol erreicht wird. Das Vakuum liegt im allgemeinen bei 5 bis 100 mbar, vorzugsweise bei 10 bis 50 mbar. Es hat sich als vorteilhaft herausgestellt, die Acylierung (Amidierung) in der Weise durchzuführen, daß man zunächst eine Mischung aus der Aminverbindung der Formel (4) und der basischen Katalysatorverbindung bereitet und diese (lösungsmittelfreie) Mischung in der Schmelze wie oben beschrieben acyliert. Das im Schritt c) erhaltene Produkt besteht im wesentlichen aus der gewünschten Verbindung der Formel (1). Mit dem erfindungsgemäßen Verfahren werden also die beschriebenen Verbindungen der Formel (1) in hoher Ausbeute und in hoher Reinheit erhalten.

Die Salze gemäß Formel (1a) werden hergestellt durch Zusammenbringen der erfindungsgemäß hergestellten Amidamine der Formel (1) mit einer anorganischen oder organischen Säure. Geeignete und bevorzugte Säuren sind oben im einzelnen genannt. Im allgemeinen wird man eine etwa 10 bis 40gew.%ige wäßrige oder wäßrig/alkoholische Lösung der Säure bereiten und in diese Lösung das pulverförmige Amidamin einrühren, in der Regel bei Raumtemperatur (etwa 20 bis 30 °C). Geeignete Alkohole sind die C₁ bis C₃-Alkanole wie Methanol, Ethanol, Propanol und/oder Isopropanol. Die wäßrigen oder wäßrig/alkoholischen Lösungen der erfindungsgemäßen Salze bestehen im wesentlichen aus 10 bis 60 Gew.-%, vorzugsweise 15 bis 40 Gew.-%, Salzverbindung und als Rest auf 100 Gew.-% Wasser oder Wasser/Alkohol. Da die wäßrigen oder wäßrig/alkoholischen Lösungen schon als solche vielfach einsetzbar sind, ist die Isolierung der Salzverbindung aus der Lösung nicht erforderlich.

Die erfindungsgemäßen Amidopropyl-N-alkylpolyhydroxyalkylamin-Verbindungen sind wertvolle und vielfach einsetzbare oberflächenaktive Verbindungen. So können sie zum Beispiel als solche oder in Mischung mit anionischen, kationischen und/oder nichtionischen Tensiden als Wasch- und Reinigungsmittel, Haar- und Körperreinigungsmittel, Textilbehandlungsmittel und dergleichen eingesetzt werden, und zwar in Form von Festprodukten, Lösungen, Dispersionen, Emulsionen, Pasten und dergleichen. Insbesondere können die Salze als Co-Tenside in Wasch- und Reinigungsmittel sowie Haar- und Körperreinigungspräparate als Schaummittel sowie zur Schaumstabilisierung eingesetzt werden. Da diese Amidamine gut biologisch abbaubar sind und aus nachwachsenden Rohstoffen hergestellt werden können, haben sie in jüngster Zeit größere Bedeutung erlangt.

Die Salze dieser Amidopropyl-N-alkyl-polyhydroxyalkylamine sind in Wasser und Mischungen aus Wasser und C₁ bis C₃-Alkoholen sehr gut löslich. Sie weisen als vorteilhafte Eigenschaft eine gute Haut- und Schleimhautverträglichkeit, wie in-vitro-Tests (Red Blood Cell Test, Zeintest) zeigen, auf, wodurch die Hautverträglichkeit der daraus hergestellten Formulierungen spürbar verbessert wird. Desweiteren zeigen die erfindungsgemäßen Verbindungen, speziell die Salze des Lauryl-/Myristylamidopropyl-N-methylglucamins ein ausgezeichnetes Schäumvermögen sowie eine schaumstabilisierende Wirkung. In Kombination mit den üblicherweise verwendeten Basistensiden (zum Beispiel Natrium-alkylethersulfate) wird eine starke viskositätserhöhende Wirkung erhalten.

Die folgenden Beispiele sollen den Gegenstand der Erfindung näher erläutert, ohne ihn darauf einzuschränken. Sofern nicht anders angegeben ist, beziehen sich die Teile und Prozentsätze auf das Gewicht.

### I. Herstellungsbeispiele

### Beispiel 1 (erste Verfahrensstufe)

### N-Methyl-N-(β-cyanoethyl)-glucamin

In einem 2-1-Vierhalskolben mit Rührer, Tropftrichter, Rückflußkühler und Thermometer werden 607 g (3,1 mol) N-Methylglucamin (NMG) in 729 g Methanol suspendiert. Das Reaktionsgemisch wird unter Rühren auf 63 °C erwärmt. Innerhalb von 30 Minuten werden 163 g (3,1 mol) Acrylnitril zugetropft. Nach Beendigung der Zugabe wird die klare Lösung weitere 3 Stunden bei 63 °C gerührt. Nach der gaschromatographischen Analyse ist der Reaktionsumsatz vollständig, so daß das Umsetzungsprodukt direkt für die Hydrierung (zweite Verfahrensstufe) eingesetzt werden kann.

### Beispiel 2 (zweite Verfahrensstufe, Hydrierung)

### N-Methyl-N-(γ-aminopropyl)-glucamin (MAPG)

In einem 2-l-Rührautoklaven werden 1500 g N-Methyl-N-(γ-cyanoethyl)-glucamin (51,4%ig in Methanol, 3,1 mol Edukt) aus Beispiel 1 und 111 g Raney-Nickel (wasserfeucht, circa 70%ig) eingefüllt. Nachdem der Autoklav verschlossen wurde, wird mit Stickstoff und Wasserstoff gespült und wieder entspannt. Anschließend werden 330 ml verflüssigtes Ammoniakgas und danach 50 bar Wasserstoff aufgepreßt. Unter Rühren wird bei 40 bis 50 bar und einer Temperatur von 25 bis 45 °C hydriert, bis kein Wasserstoff mehr aufgenommen wird (circa 3 Stunden). Bei 40 °C wird über eine Druckfilterpresse der Katalysator abfiltriert bei gleichzeitiger Entspannung des Systems. Die methanolische Lösung des hydrierten Produktes wird am Rotationsverdampfer bei 70 °C und im Wasserstrahlvakuum eingeengt. Es werden 742 g N-Methyl-N-(γ-aminopropyl)-glucamin - entsprechend einer Ausbeute von 95 % der Theorie - in Form eines farblosen Pulvers erhalten.
- Schmelzpunkt:: 106 °C
- Zusammensetzung (¹³C-NMR):: 96 Mol-% MAPG
2 Mol-% NMG
2 Mol-% Di-NMG

Alternativ zur beschriebenen Hydrierung kann auch wie folgt verfahren werden: Nachdem der Hydriervorgang beendet ist, läßt man innerhalb 1 Stunde den Katalysator absetzen und drückt die überstehende Lösung auf die Druckfilterpresse, um geringe Mengen Katalysatorreste abzufiltrieren. Die weitere Aufarbeitung erfolgt so, wie in Beispiel 2 beschrieben ist. Auf diese Weise kann für Folgeansätze ohne weitere Katalysatorzugabe durch jeweiliges Zupumpen der Reaktanten (nach jedem Entleeren des Autoklaven) eine Serie von mindestens 12 Folgeansätzen gefahren werden, ohne daß die Katalysatoraktivität erschöpft ist, bei gleichbleibender Qualität des Endproduktes.

### Beispiel 3 (Amidierung)

### Lauryl-/Myristylamidopropyl-N-methylglucamin (LAP-NMG)

In einem 2-l-Vierhalskolben mit Rührer, Tropftrichter, Destillationsaufsatz und Thermometer werden 300 g (1,1 mol) MAPG aus Beispiel 2 bei 100 °C aufgeschmolzen. Es werden 4,5 g (0,056 mol) 50%ige Natronlauge zugegeben und auf 130 °C erhitzt. Nach 15 Minuten bei 130 °C und Normaldruck und 15 Minuten bei einem Vakuum von 16 mbar wird das gesamte Wasser abdestilliert. Innerhalb 20 Minuten werden bei 100 °C 242 g (1,1 mol) Laurin-/Myristinsäuremethylester zugetropft. In 4 Stunden wird bei 100 °C und einem Vakuum von 16 mbar das entstehende Methanol abdestilliert. Es werden in nahezu quantitativer Ausbeute 508 g einer hell-bernsteinfarbenen Schmelze erhalten, die beim Abkühlen erstarrt. Nach Zerkleinern in einem Mixer entsteht ein farbloses Pulver mit einem Schmelzbereich von 93 bis 99 °C, das nach ¹³C-NMR-Spektroskopie aus 96%igem LAP-NMG besteht.

### Beispiel 4 (Amidierung)

### Palmityl-/Stearylamidopropyl-N-methylglucamin (PAP-NMG)

In entsprechender Weise, wie beim Beispiel 3 beschrieben ist, werden aus 300 g (1,1 mol) MAPG aus Beispiel 2 durch Umsetzung mit Palmitin-/Stearinsäuremethylester (nach Zugabe des Katalysators und Behandlung wie beschrieben) bei 120 °C nach vierstündiger Reaktionszeit bei einem Vakuum von 16 mbar 572 g PAP-NMG in praktisch quantitativer Ausbeute erhalten mit einer Reinheit von 94 Mol-% (¹³C-NMR). Nach dem Zerkleinern der Schmelze entsteht ein farbloses Pulver mit einem Schmelzbereich von 101 bis 104,5 °C.

**Tabelle 1**

| [Zusammensetzung der Endprodukte (¹³C-NMR)] | | |
|---|---|---|
| Beispiel | 3 | 4 |
| Fettalkyl-amidopropyl-NMG | 96 Mol-% | 94 Mol-% |
| Di-NMG-amin | 2 Mol-% | 2 Mol-% |
| N-Methylglucamin | 2 Mol-% | 2 Mol-% |
| Esteramid | --- | 2 Mol-% |

### Beispiel 5

### Lauryl-/Myristylamidopropyl-N-methylglucaminhydrochlorid (LAP-NMG·HCl)-Lösung

In einem 2-l-Dreihalskolben mit Rührer, Rückflußkühler und Thermometer werden 900 ml Wasser vorgelegt und unter Rühren 17,9 g konzentrierte Salzsäure (37%ig, 0,182 mol) und 82,1 g (0,182 mol) LAP-NMG aus Beispiel 3 zugegeben und 1 Stunde auf 40 bis 50 °C erwärmt. Es wird 1 1 einer 10%igen klaren, farblosen, wäßrigen LAP-NMG-Hydrochlorid-Lösung erhalten mit einem pH-Wert von 5,5 bis 6,5.

### Beispiel 6

### Lauryl-/Myristylamidopropyl-N-methylglucamin-lactat-Lösung (LAP-NMG-Lactat-Lösung)

In entsprechender Weise, wie in Beispiel 3 beschrieben ist, wird aus 900 ml Wasser, 21,5 g (0,174 mol) Milchsäure und 78,5 g (0,174 mol) LAP-NMG (aus Beispiel 3) 1 1 einer 10%igen klaren, farblosen, wäßrigen Lactat-Lösung mit einem pH-Wert von 6,5 bis 7 erhalten.

### Beispiel 7

### Palmityl-/Stearylamidopropyl-N-methylglucaminhydrochlorid (PAP-NMG·HCl)-Lösung

In entsprechender Weise wird aus 900 ml Wasser, 16,2 g (0,164 mol) konzentrierte Salzsäure und 83,8 g (0,164 mol) Palmityl-/Stearylamidopropyl-N-methylglucamin (aus Beispiel 4) 1 l einer 10%igen Lösung von PAP-NMG·HCl erhalten mit einem pH-Wert von 5.

### Beispiel 8

### Palmityl-/Stearylamidopropyl-N-methylglucamin-lactat-Lösung (PAP-NMG-Lactat-Lösung)

In entsprechender Weise wird aus 900 ml Wasser, 19,4 g (0,157 mol) Milchsäure und 80,6 g (0,157 mol) Palmityl-/Stearylamidopropyl-N-methylglucamin (aus Beispiel 4) 1 l einer 10%igen Lactat-Lösung erhalten mit einem pH-Wert von 6,5.

## Patentansprüche

1. Verfahren zur Herstellung reiner Amidopropyl-N-alkyl-polyhydroxyalkylamine der nachstehenden Formel (1) 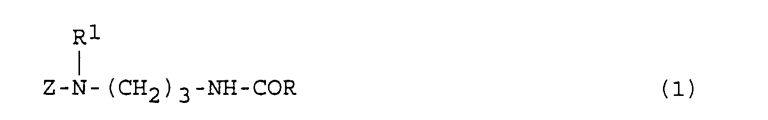 worin bedeuten
Z einen linearen Polyhydroxykohlenwasserstoffrest mit mindestens 2 und maximal 13 gegebenenfalls oxalkylierten Hydroxylgruppen,
R¹ einen C₁ bis C₇-Alkylrest oder C₂ bis C₄-Hydroxyalkylrest und
COR einen aliphatischen Acylrest mit 6 bis 22 Kohlenstoffatomen,
und deren Salze von einer anorganischen oder organischen Säure, gekennzeichnet durch
a) Umsetzung einer Verbindung der Formel (2) 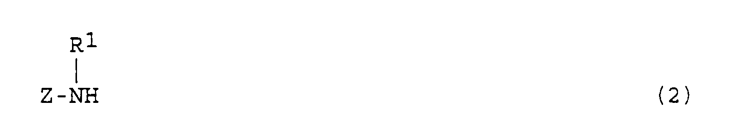 in der Z und R¹ die genannten Bedeutungen haben,
mit Acrylnitril (CH₂=CH-CN) in einem polaren Lösungsmittel bei einer Temperatur von 50 bis 100 °C zur Bildung einer Verbindung der Formel (3) 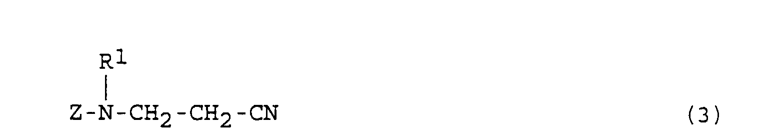 in der Z und R¹ die genannten Bedeutungen haben,
b) Umsetzung des im Schritt a) erhaltenen Produktes mit Wasserstoff in Gegenwart von Ammoniak und einem Hydrierkatalysator bei einer Temperatur von 20 bis 80 °C und einem Wasserstoffdruck von 10 bis 80 bar zur Bildung einer Verbindung der Formel (4) 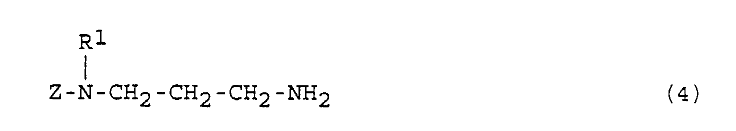 in der Z und R¹ die genannten Bedeutungen haben,
und
c) Monoacylierung des im Schritt b) erhaltenen Produktes in der Schmelze mit einer vom Acylrest COR in Formel (1) abgeleiteten Säure oder Säurederivat in Gegenwart eines alkalischen Katalysators bei einer Temperatur von 80 bis 130 °C zur Bildung einer Verbindung der genannten Formel (1).

2. Verfahren nach Anspruch 1, gekennzeichnet durch
a) Umsetzung einer Verbindung der Formel (2) mit Acrylnitril im Molverhältnis von etwa 1 : 1 in Methanol, Ethanol, Propanol oder Isopropanol als Lösungsmittel bei einer Temperatur von 50 bis 100 °C,
b) Umsetzung des im Schritt a) erhaltenen Produktes mit Wasserstoff in Gegenwart von 1 bis 10 mol Ammoniak pro mol Nitrilverbindung der Formel (3) und von Raney-Nickel als Hydrierkatalysator bei einer Temperatur von 20 bis 80 °C und einem Wasserstoffdruck von 10 bis 80 bar und
c) Monoacylierung des im Schritt b) erhaltenen Produktes in der Schmelze mit einem vom Acylrest COR in Formel (1) abgeleiteten Fettsäure-C₁ bis C₄-alkylester in Gegenwart von einem Alkalimetallhydroxid oder einem Alkalimetall-C₁ bis C₄-alkoholat als Katalysator bei einer Temperatur von 80 bis 130 °C.

3. Verfahren nach Anspruch 1, gekennzeichnet durch
a) Umsetzung einer Verbindung der Formel (2) mit Acrylnitril im Molverhältnis von etwa 1 : 1 in Methanol als Lösungsmittel bei einer Temperatur von 60 bis 90 °C,
b) Umsetzung des im Schritt a) erhaltenen Produktes mit Wasserstoff in Gegenwart von 3 bis 6 mol Ammoniak pro mol Nitrilverbindung der Formel (3) und von Raney-Nickel als Hydrierkatalysator bei einer Temperatur von 30 bis 60 °C und einem Wasserstoffdruck von 30 bis 60 bar und
c) Monoacylierung des im Schritt b) erhaltenen Produktes in der Schmelze mit einem vom Acylrest COR in Formel (1) abgeleiteten Fettsäuremethylester in Gegenwart von einem Alkalimetallhydroxid oder einem Alkalimetallmethylat als Katalysator bei einer Temperatur von 90 bis 120 °C.

## Claims

1. A process for the preparation of a pure amidopropyl-N-alkyl-polyhydroxyalkylamine of the following formula (1) 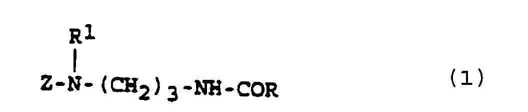 in which
Z is a linear polyhydroxyhydrocarbon radical having at least 2 and at most 13 free or oxyalkylated hydroxyl groups,
R¹ is a C₁ to C₇-alkyl radical or C₂ to C₄-hydroxyalkyl radical and
COR is an aliphatic acyl radical having 6 to 22 carbon atoms,
or a salt thereof from an inorganic or organic acid, which comprises
a) reaction of a compound of the formula (2) 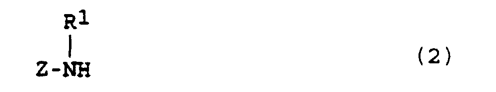 in which Z and R¹ have the meanings given, with acrylonitrile (CH₂=CH-CN) in a polar solvent at a temperature of 50 to 100°C to form a compound of the formula (3) 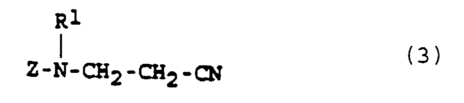 in which Z and R¹ have the meanings given,
b) reaction of the product obtained in step a) with hydrogen in the presence of ammonia and a hydrogenation catalyst at a temperature of 20 to 80°C under a hydrogen pressure of 10 to 80 bar, to form a compound of the formula (4) 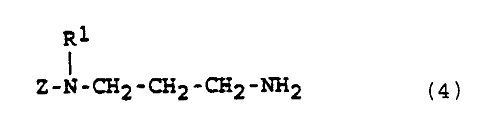 in which Z and R¹ have the meanings given,
and
c) monoacylation of the product obtained in step b) in the melt with an acid or acid derivative derived from the acyl radical COR in formula (1) in the presence of an alkaline catalyst at a temperature of 80 to 130°C to form a compound of the formula (1) mentioned.

2. The process as claimed in claim 1, which comprises
a) reaction of a compound of the formula (2) with acrylonitrile in a molar ratio of about 1 : 1 in methanol, ethanol, propanol or isopropanol as the solvent at a temperature of 50 to 100°C,
b) reaction of the product obtained in step a) with hydrogen in the presence of 1 to 10 mol of ammonia per mole of nitrile compound of the formula (3) and of Raney nickel as the hydrogenation catalyst at a temperature of 20 to 80°C under a hydrogen pressure of 10 to 80 bar and
c) monoacylation of the product obtained in step b) in the melt with a fatty acid C₁ to C₄-alkyl ester derived from the acyl radical COR in formula (1) in the presence of an alkali metal hydroxide or an alkali metal C₁ to C₄-alcoholate as the catalyst at a temperature of 80 to 130°C.

3. The process as claimed in claim 1, which comprises
a) reaction of a compound of the formula (2) with acrylonitrile in a molar ratio of about 1 : 1 in methanol as the solvent at a temperature of 60 to 90°C,
b) reaction of the product obtained in step a) with hydrogen in the presence of 3 to 6 mol of ammonia per mole of nitrile compound of the formula (3) and of Raney nickel as the hydrogenation catalyst at a temperature of 30 to 60°C under a hydrogen pressure of 30 to 60 bar and
c) monoacylation of the product obtained in step b) in the melt with a fatty acid methyl ester derived from the acyl radical COR in formula (1) in the presence of an alkali metal hydroxide or an alkali metal methylate as the catalyst at a temperature of 90 to 120°C.

## Revendications

1. Procédé pour la préparation d'amidopropyl-N-alkylpolyhydroxyalkylamines pures de la formule suivante (1) 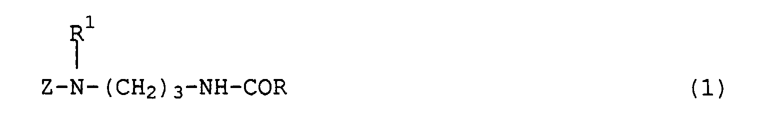 dans laquelle
Z représente un groupe polyhydroxyhydrocarboné ayant au moins 2 et au maximum 13 groupes hydroxyle éventuellement alcoxylés,
R¹ représente un groupe alkyle en C₁ à C₇ ou un groupe hydroxyalkyle en C₂ à C₄ et
COR représente un groupe acyle aliphatique ayant 6 à 22 atomes de carbone,
et leurs sels avec un acide inorganique ou organique, caractérisé par les opérations de
a) réaction d'un composé de formule (2) 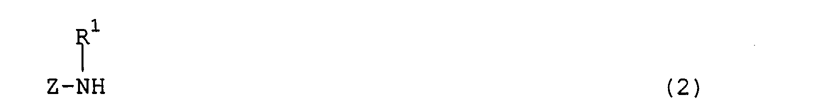 dans laquelle Z et R¹ ont les significations indiquées,
avec l'acrylonitrile (CH₂=CH-CN) dans un solvant polaire à une température de 50 à 100°C pour former un composé de formule (3) 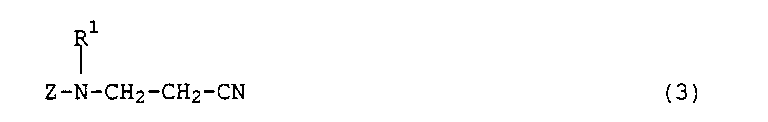 dans laquelle Z et R¹ ont les significations indiquées,
b) réaction du produit obtenu à l'étape a) avec l'hydrogène en présence d'ammoniac et d'un catalyseur d'hydrogénation à une température de 20 à 80°C et sous une pression d'hydrogène de 10 à 80 bar pour former un composé de formule (4) 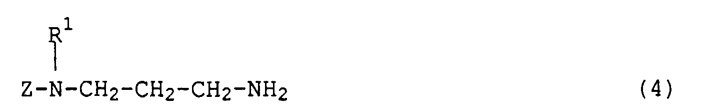 dans laquelle Z et R¹ ont les significations indiquées,
et
c) monoacylation du produit obtenu à l'étape b) à l'état fondu avec un acide dérivant du groupe acyle COR dans la formule (1) ou d'un dérivé d'acide en présence d'un catalyseur alcalin à une température de 80 à 130°C pour former un composé de la formule citée (1).

2. Procédé selon la revendication 1, caractérisé par les opérations de
a) réaction d'un composé de formule (2) avec l'acrylonitrile dans un rapport molaire d'environ 1 : 1 dans le méthanol, l'éthanol, le propanol ou l'isopropanol comme solvant à une température de 50 à 100°C,
b) réaction du produit obtenu à l'étape a) avec l'hydrogène en présence de 1 à 10 mol d'ammoniac par mol de composé nitrile de formule (3) et de nickel de Raney comme catalyseur d'hydrogénation à une température de 20 à 80°C et sous une pression d'hydrogène de 10 à 80 bar et
c) monoacylation du produit obtenu à l'étape b) à l'état fondu avec un ester d'alkyle en C₁ à C₄ avec un acide gras dérivant du groupe acyle COR dans la formule (1) en présence d'un hydroxyde de métal alcalin ou d'un alcoolate de métal alcalin en C₁ à C₄ comme catalyseur à une température de 80 à 130°C.

3. Procédé selon la revendication 1, caractérisé par les opérations de
a) réaction d'un composé de formule (2) avec l'acrylonitrile dans un rapport molaire d'environ 1 : 1 dans le méthanol comme solvant à une température de 60 à 90°C,
b) réaction du produit obtenu à l'étape a) avec l'hydrogène en présence de 3 à 6 mol d'ammoniac par mol de composé nitrile de formule (3) et de nickel de Raney comme catalyseur d'hydrogénation à une température de 30 à 60°C et sous une pression d'hydrogène de 30 à 60 bar et
c) monoacylation du produit obtenu à l'étape b) à l'état fondu avec un ester méthylique d'acide gras dérivant du groupe acyle COR dans la formule (1) en présence d'un hydroxyde de métal alcalin ou d'un méthylate de métal alcalin comme catalyseur à une température de 90 à 120°C.
